# EUROPÄISCHE PATENTSCHRIFT

(11) **EP 3 104 770 B1**
(45) Veröffentlichungstag und Bekanntmachung des Hinweises auf die Patenterteilung: **26.06.2024**
(21) Anmeldenummer: 15706188.8
(22) Anmeldetag: 13.02.2015
(51) Int. Cl.: A61B 5/02, A61M 1/36, A61B 5/00, G01N 27/12, G01M 3/04, G01M 3/16, A61M 5/14, A61M 5/168, A61M 5/158

(54) **ABDECKUNGSEINRICHTUNG UND VORRICHTUNG ZUR ÜBERWACHUNG DER KONNEKTOREN EINES SCHLAUCHSYSTEMS**
COVER UNIT AND DEVICE FOR MONITORING THE CONNECTORS OF A TUBING SYSTEM
REVÊETEMENT ET DISPOSITIF POUR LA SURVEILLANCE DES CONNECTEURS D'UN SYSTÈME DE TUYAU

(30) Priorität: 14.02.2014 DE 102014001898
(43) Veröffentlichungstag der Anmeldung: 21.12.2016
(73) Patentinhaber: Fresenius Medical Care Deutschland GmbH, 61352 Bad Homburg (DE)
(72) Erfinder: SCHRÖRS, Alexander, 60389 Frankfurt (DE)
(74) Vertreter: Breuninger, Marcus
(86) Internationale Anmeldenummer: PCT/EP2015/053146
(87) Internationale Veröffentlichungsnummer: WO 2015/121440

(56) Entgegenhaltungen:
- EP-A1- 2 604 303
- EP-A1- 2 604 303
- WO-A1-99/24145
- WO-A1-99/24145
- WO-A1-2013/047855
- WO-A1-2013/047855
- WO-A1-2014/008980
- WO-A1-2014/008980
- WO-A1-2014/008991
- WO-A1-2014/008991
- JP-A- 2004 177 120
- JP-A- 2004 177 120
- US-A- 3 625 209
- US-B1- 6 445 304
- US-B1- 6 445 304

## Beschreibung

### Darstellung der Erfindung

Die Erfindung betrifft eine Abdeckungseinrichtung eines an einem Patienten anbringbaren Schlauchsystems, mit dem Undichtigkeiten detektiert werden können.

Feuchtigkeitssensoren in Form von Pflastern zur Überwachung des Gefäßzugangs von Patienten sind z.B. bekannt aus der WO 2006/008866 A1 und der US 6,445,304 B1. In der EP 2 604 303 wird ein Detektor zur Überwachung einer Punktionsstelle beschrieben, der eine Sensoreinheit aufweist, die ohne direkte Kopplung an eine externe Stromquelle arbeitet. Ebenso im Stand der Technik bekannt ist ein Feuchtigkeitssensor in Form einer Manschette, die um das Schlauchsystem gelegt werden kann.

Im Gebiet der Medizintechnik sind verschiedene Einrichtungen bekannt, mit denen dem Patienten über einen Gefäßzugang, z.B einen Katheter, und über ein Schlauchsystem Flüssigkeiten entnommen und auch wieder zugeführt werden.

Ein Beispiel sind extrakorporale Blutbehandlungsvorrichtungen, wie z.B. Dialysevorrichtungen oder Zellseparatoren, die einen Zugang zum Gefäßsystem des Patienten erforderlich machen, um einen extrakorporalen Blutkreislauf herzustellen. Bei solchen Behandlungen ist es üblich, dass der Gefäßzugang und das benachbarte Schlauchsystem in eine Schutzhülle, im Folgenden genannt textiles Feld, eingeschlagen werden. Damit soll eine möglichst keimfreie Umgebung für den Gefäßzugang und das Schlauchsystem bewahrt werden. Dieses textile Feld wird teilweise von Herstellern von Blutschlauchsystemen in Form eines Tuches oder als Gaze-Feld mit dem Blutschlauchsystem mitgeliefert. Es wird vom Pflegepersonal als Unterlage und Umhüllung für das Schlauchsystem verwendet.

Sind der Gefäßzugang und das Schlauchsystem von dem sterilen Feld bedeckt, so kann dies dazu führen, dass Leckagen am Gefäßzugang oder an Luer-Lock-Konnektoren des Schlauchsystems vom Pflegepersonal nicht frühzeitig erkannt werden können. Diese Leckagen können durch ein Herausrutschen der Kanüle aus dem Gefäß des Patienten oder bei einem Lösen der Verbindung der Luer-Lock-Konnektoren auftreten. Gerade bei extrakorporalen Blutbehandlungen können wegen der hohen Flüsse auch kleine Undichtigkeiten zu unerwünschten Blutverlusten führen. In festgelegten Zeitabständen muss daher die Abdeckung entfernt und der Zugang kontrolliert werden.

Im Stand der Technik sind zur Überwachung des Gefäßzugangs verschiedene Vorrichtungen unterschiedlicher Ausbildung bekannt. Die bekannten Überwachungsvorrichtungen greifen im Allgemeinen auf die standardmäßig in den Blutbehandlungsvorrichtungen vorhandenen Sicherheitssysteme zurück, die bei einem nicht ordnungsgemäßen Gefäßzugang eine sofortige Unterbrechung des extrakorporalen Blutkreislaufs auslösen.

Solche Vorrichtungen zur Überwachung können z.B. Vorrichtungen zur Erkennung von Feuchtigkeit oder Vorrichtungen mit optischen Sensoren sein, die den Austritt von Blut aus der Punktionsstelle oder Verbinden des Schlauchsystems erkennen. Die Feuchtigkeitssensoren sind z.B. als Pad ausgebildet, der auf die Haut des Patienten aufgelegt wird, oder als Manschette, die um die Konnektoren des Schlauchsystems gebunden wird.

Diese bekannten Vorrichtungen sind lediglich zur kleinflächigen Anwendung direkt an der Punktionsstelle ausgebildet und können keine Abdeck- und Schutzfunktion erfüllen. Zudem kann es bei diesen bekannten Vorrichtungen zu Fehlalarmen kommen, wenn beim Anschließen der Blutschläuche oder bei Manipulationen der Konnektoren durch das Pflegepersonal kleinere Mengen Flüssigkeit aus dem Schlauchende austreten.

Aufgabe der vorliegenden Erfindung ist es eine Vorrichtung zur Abdeckung eines Schlauchsystems zur Verfügung zu stellen, die ein frühzeitiges Erkennen einer Leckage ermöglicht, wobei diese Vorrichtung unabhängig von der Ausführungsform des Schlauchsystems sein soll. Dabei sollen Fehlalarme bei Leckagen, die bei Arbeitsvorgängen an dem Schlauchsystem auftreten können, vermieden werden.

Nach der Lehre der vorliegenden Erfindung wird diese Aufgabe gelöst durch eine Abdeckungseinrichtung nach Anspruch 1 und eine damit verbundene Vorrichtung zur Überwachung eines Zugangs eines Patienten nach Anspruch 13. Vorteilhafte Ausgestaltungen sind Gegenstand der Unteransprüche.

Die erfindungsgemäße Abdeckungseinrichtung weist ein Flächengebilde und einen Flüssigkeitssensor zur Detektion einer Leckage am Gefäßzugang oder am Schlauchsystem während einer Blutbehandlung auf.

Das Flächengebilde weist weiterhin eine Knickkante auf. Bei der Faltung des Flächengebildes entlang der Knickkante bildet sich eine Faltentasche. Die Faltentasche ist der Bereich der Abdeckungseinrichtung, in dem zwei Lagen des Flächengebildes übereinander liegen. Die Faltentasche weist eine untere und eine obere Teilfläche auf, wobei die untere Teilfläche, die dem Patienten nähere Teilfläche ist.

Ganz allgemein soll die untere Anordnung immer eine dem Patienten nähere Orientierung bezeichnen als die obere Anordnung.

Der Flüssigkeitssensor ist derart auf dem Flächengebilde angeordnet, dass er nach Faltung des Flächengebildes entlang der Knickkante in der sich ergebenden Faltentasche angeordnet ist. Zumindest ein Teil des Flüssigkeitssensors kann auf der unteren Teilfläche der Faltentasche angeordnet sein.

Ein Vorteil der erfindungsgemäßen Abdeckungseinrichtung ist die Erleichterung der Versorgung eines Patienten während einer extrakorporalen Blutbehandlung und die Erhöhung der Sicherheit der Behandlung.

In einer weiteren Ausführungsform weist das Flächengebilde eine zweite Knickkante auf, die weitgehend parallel zur ersten Knickkante verläuft und in Gegenrichtung zur ersten Knickkante ausgebildet ist. Bei der Faltung des Flächengebildes entlang der beiden Knickkanten ergibt sich eine Zickzackfaltung mit einer ersten und einer zweiten Faltentasche, die sich in entgegengesetzte Richtungen öffnen.

Durch die Zickzackfaltung kann die Faltung und Entfaltung der Faltentaschen durch seitliches Ziehen bzw. Zusammenschieben an dem Flächengebilde erfolgen.

In einer weiteren Ausführungsform weist das Flächengebilde zumindest vier Knickkanten auf, wobei benachbarte Knickkanten stets in entgegengesetzte Richtungen ausgebildet sind und sich bei der Faltung eine Zickzack-Faltung mit zumindest drei Faltentaschen ergibt.

Der Flüssigkeitssensor befindet sich vorzugsweise in der untersten Faltentasche.

In einer Ausführungsform liegt der Flüssigkeitssensor höchstens auf einer Knickkante des Flächengebildes. Dadurch wird das Risiko der Beschädigung des Flüssigkeitssensors durch das Knicken minimiert.

In einer Ausführungsform sind die Teilflächen der Faltentaschen breiter als das abzudeckende Schlauchsystem. Weist die Abdeckungseinrichtung mehr als eine Faltentasche auf, so ist die Breite der Teilfläche der Faltentasche durch den Abstand zwischen zwei Knickkanten definiert. Die Breite der Teilfläche der Faltentasche kann zwischen 5 und 15 cm, bevorzugt zwischen 8 und 12 cm, betragen.

In einer Ausführungsform kann das Flächengebilde ein textiles Gewebe sein, eine saugfähige Folie und/oder ein Vlies, welche einen angenehmen Hautkontakt bieten.

In einer weiteren Ausführungsform kann das Flächengebilde auf einer Seite feuchtigkeitsundurchlässig sein, womit ein Durchdringen der Flüssigkeit durch ein saugfähiges Gewebe und so die Auslösung von Fehlalarmen verhindert.

In einer alternativen Ausführungsform kann das Flächengebilde feuchtigkeitsundurchlässig, z.B. ein beschichtetes Papier sein, womit die erfindungsgemäße Vorrichtung einfach und zuverlässig hergestellt werden kann.

Das Flächengebilde kann sterilisierbar sein.

Das Flächengebilde kann zumindest auf einer Seite steril sein.

Das Flächengebilde kann sterilisiert sein.

In einer Ausführungsform kann das Flächengebilde zwischen 400 und 600 mm lang und zwischen 350 und 550 mm breit sein.

In einer Ausführungsform kann der Flüssigkeitssensor zwischen 100 und 200 mm lang und zwischen 100 und 200 mm breit sein.

Das Flächengebilde kann Markierungen aufweisen, die dem Benutzer anzeigen, wie das Flächengebilde in Bezug zum Gefäßzugang anzuordnen ist. Diese Markierungen können dem Benutzer anzeigen, welche Position möglichst nahe an den Ort des Gefäßzugangs anzuordnen ist. Weiterhin können Markierungen dem Benutzer anzeigen, wo und in welcher Richtung das Flächengebilde durch Auseinanderziehen und Zusammenklappen gefaltet und entfaltet wird.

Diese Markierung kann in Form von Pfeilen ausgeführt sein.

In einer Ausführungsform der Abdeckungseinrichtung sind der Flüssigkeitssensor und das Flächengebilde fest miteinander verbunden. Der Flüssigkeitssensor kann in das Flächengebilde integriert sein. Der Flüssigkeitssensor kann in einer alternativen Ausführungsform an dem Flächengebilde befestigt sein. Der Flüssigkeitssensor kann auf das Flächengebilde aufgenäht oder aufgeklebt sein. Die Position des Flüssigkeitssensors sollte mittig am Rand des Flächengebildes liegen. Damit kann das Flächengebilde mit dem Flüssigkeitssensor möglichst nah an die Austrittsöffnung des Katheters herangeschoben werden. Möglich ist auch ein Ausschnitt im Flächengebilde, an dessen Ende sich der Flüssigkeitssensor befindet, so dass das Flächengebilde den Gefäßzugang von allen Seiten umschließt.

Der Flüssigkeitssensor kann eine elektrisch leitende Struktur aufweisen. Die elektrisch leitende Struktur kann eine oder mehrere Leiterbahnen aufweisen.

Die elektrisch leitende Struktur kann auf ein Gewebe oder ein Vlies aufgedruckt sein.

Die elektrisch leitende Struktur kann in das Gewebe eingewebt sein.

Zur elektrischen Kontaktierung des Flüssigkeitssensors weist die erfindungsgemäße Abdeckungsvorrichtung elektrische Anschlusskontakte auf. Die Anzahl der Anschlusskontakte richtet sich nach der Ausbildung des Flüssigkeitssensors. Alternativ können auch elektrische Verbindungsleitungen aus dem Flüssigkeitssensor herausgeführt werden, die mit einer Vorrichtung zur Überwachung des Gefäßzugangs eines Patienten verbunden werden können.

In einer besonderen Ausführungsform der erfindungsgemäßen Abdeckungseinrichtung ist am Rand des Flüssigkeitssensors eine Barriere aus einem saugfähigen Material ohne Messfunktion abgebracht ist, um ein seitliches Eindringen von Flüssigkeit in die Faltung zu vermeiden.

Die Barriere soll dabei keinen Kontakt zum Flüssigkeitssensor haben. Der Abstand zwischen dem Flüssigkeitssensor kann z.B. zwischen o,5 und 1 cm betragen.

Dabei kann die Barriere innerhalb der Faltentasche angeordnet sein, d.h. im gefalteten Zustand des Flächengebildes wird die Barriere durch die obere Teilfläche der Faltentasche abgedeckt, oder die Barriere ist außerhalb der Faltentasche in Anschluss an die Knickkante der Faltentasche angeordnet.

Die erfindungsgemäße Vorrichtung zur Überwachung der Konnektoren eines Schlauchsystems, insbesondere zur Überwachung der Konnektoren des Blutschlauchsystems bei einer extrakorporalen Blutbehandlung, verfügt über die erfindungsgemäße Abdeckvorrichtung zur Detektion von Feuchtigkeit, nämlich dem Flächengebilde mit dem Flüssigkeitssensor, die an die Überwachungsvorrichtung angeschlossen wird. Die Überwachungsvorrichtung kann einen akustischen und/oder optischen und/oder taktilen Alarm auslösen, wenn Feuchtigkeit detektiert wird. Auch kann ein Steuersignal für einen Eingriff in die Steuerung der Einrichtung erzeugt werden, mit der über eine Schlauchleitung eine Flüssigkeit dem Patienten zugeführt und/oder eine Flüssigkeit von dem Patienten abgeführt wird.

### Kurze Beschreibung der Zeichnungen

Im Folgenden werden weitere Ausgestaltungen und Vorteile der Erfindung anhand eines Ausführungsbeispiels und einiger Figuren näher erläutert.

Dabei zeigt
Figur 1: einen Querschnitt durch ein Flächengebilde, der Flüssigkeitssensor wird durch die Faltung des Flächengebildes geschützt, Blutschlauchsystem liegt auf den Flächengebilde über dem Flüssigkeitssensor.
Figur 2: eine Draufsicht auf die Anordnung in Figur 1, Blutschlauchsystem auf Faltung angeordnet. Flüssigkeitssensor in Faltung (gestrichelt) in Faltung.
Figur 3: einen Querschnitt durch Flächengebilde wie in Figur 1, Blutschlauchsystem auf dem Flüssigkeitssensor angeordnet, durch Faltung abgedeckt.
Figur 4: Aufsicht auf Anordnung in Figur 3, Blutschlauchsystem (gestrichelt) durch Faltung abgedeckt.
Figur 5: Querschnitt durch ein Flächengebilde mit Flüssigkeitssensor, auseinandergefaltet, 5 Faltungen 4-4' mit Knickrichtungen der Faltung angedeutet.
Figur 6: Querschnitt durch Flächengebilde mit 5 Knickkanten
Figur 7: Aufsicht auf Flächengebilde mit 5 Faltungen mit Flüssigkeitssensor aufgeklappt mit Leiterbahnen und Anschlusskontakt.
Figur 8: Aufsicht auf Flächengebilde mit Flüssigkeitssensor, auseinandergefaltet mit seitlicher Barriere.
Figur 9: Querschnitt durch Flächengebilde mit Flüssigkeitssensor und mit seitlicher Barriere, zusammengefaltet.
Figur 10: Querschnitt durch Flächengebilde mit Flüssigkeitssensor und mit seitlicher Barriere, zusammengefaltet, mit Blutschlauchsystem.

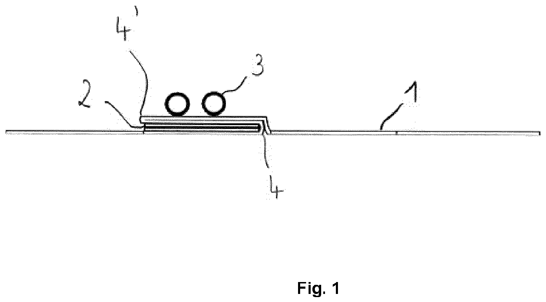

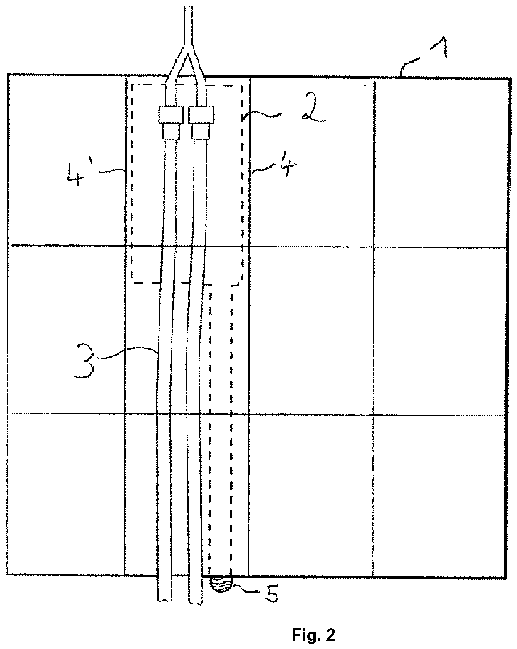

In Figur 1 ist eine erfindungsgemäße Abdeckungsvorrichtung gezeigt, aufweisend ein Flächengebilde 1, das in diesem Ausführungsbeispiel ein beschichtetes Papier ist, das vorzugsweise sterilisierbar, flexibel und wasserabweisend ist. In dem beschichteten Papier ist eine erste Knickkante 4 eingebracht, die ein Umfalten des Papiers 1 nach oben ermöglicht, so dass ein umgefalteter Teil des beschichteten Papiers, wie in Fig. 1 dargestellt, nach der Knickkante 4' planparallel auf dem restlichen Teil des beschichteten Papiers zu liegen kommt und eine Faltentasche bildet. Mit "nach oben" ist in diesem Zusammenhang in Bezug auf die Anordnung der Abdeckungsvorrichtung in Fig. 1 gemeint. Ein Flüssigkeitssensor 2 ist in diesem Ausführungsbeispiel auf dem beschichteten Papier 1 aufgedruckt und wird durch die Knickkante 4 ungefähr in der Mitte geschnitten. Daher wird eine Hälfte des Sensors 2 beim Umfalten des beschichteten Papiers auf die andere Hälfte des Sensors 2 gefaltet. Das beschichtete Papier weist des Weiteren eine zweite Knickkante 4' auf, an der das beschichtete Papier in Gegenrichtung umfaltbar ist. Nach dieser zweiten Umfaltung bildet sich eine zweite Faltentasche, eine Art Deckel, der aus einer Doppellage beschichteten Papiers besteht, das den Sensor 2 vollständig bedeckt. In dem in Fig. 1 und Fig. 2 gezeigten Anordnungen liegt die Abdeckungsvorrichtung zu Behandlungsbeginn auf dem Patienten auf, vorzugsweise in unmittelbarer Nähe zu einem Gefäßzugang, der beispielsweise für eine Dialysebehandlung gelegt ist. Ein Schlauchsystem zum Anschluss an den Gefäßzugang wird dann während eines Anschließens von Konnektoren auf der obersten Faltentasche bzw. dem Deckel abgelegt. In diesem Schritt eventuell austretende Flüssigkeit kann die Flüssigkeitssperre nicht durchdringen. Das Flächengebilde kann auch so ausgeführt sein, dass die Feuchtigkeit von dem Gewebe des Textils aufgesaugt wird. Der Flüssigkeitssensor 2 befindet sich unter dem Deckel in einer sich durch die Faltung gebildeten Faltentasche und kommt daher nicht in Kontakt mit der Flüssigkeit, so dass kein Fehlalarm ausgelöst wird.

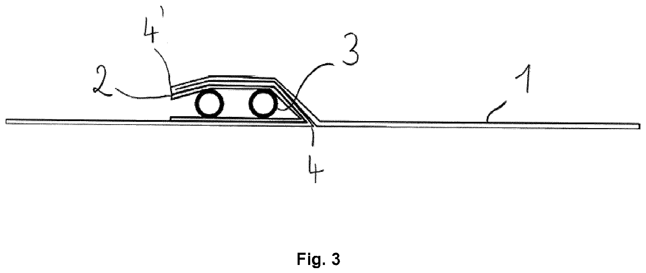

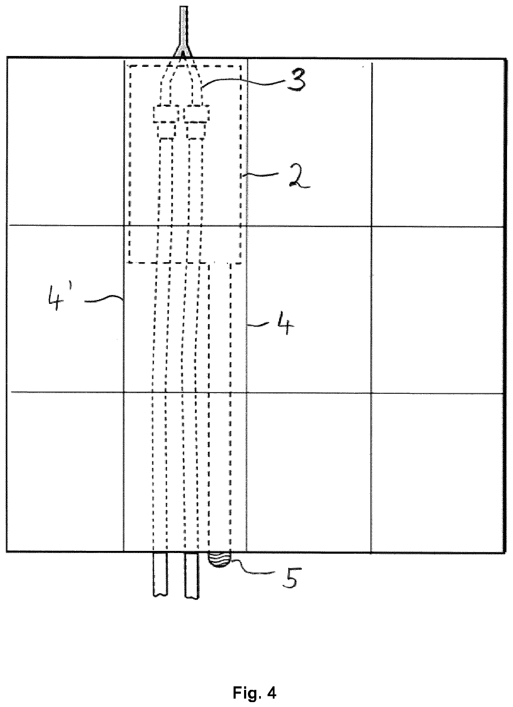

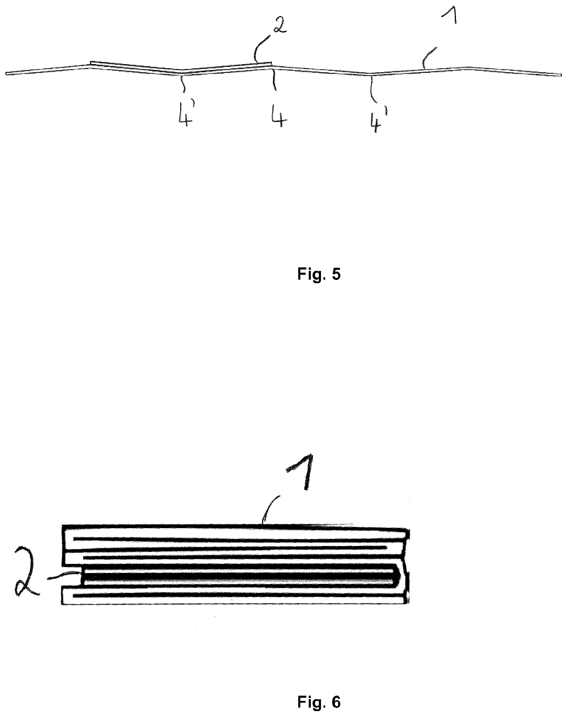

Sind alle Anschlüsse angebracht, kann die Faltung durch Auseinanderziehen des beschichteten Papiers aufgefaltet werden, wodurch der Sensor exponiert wird. Dies ist in Fig. 5 dargestellt. Das Schlauchsystem kann nun im Bereich des Flüssigkeitssensors auf das beschichtete Papier gelegt werden. Anschließend kann die Faltung wieder zugeklappt werden. Der sich ergebende Zustand ist in Fig. 3 im Querschnitt und in Fig. 4 in Draufsicht dargestellt. Eventuell nun auftretende Leckagen an den Konnektoren oder an dem Gefäßzugang können von dem Flüssigkeitssensor detektiert werden. Gleichzeitig wird das Schlauchsystem von dem Deckel nach oben hin vor Kontamination geschützt.

In Fig. 2 und Fig. 4 zu erkennen ist ein elektrischer Anschluss 5, der ermöglicht die erfindungsgemäße Abdeckungseinrichtung an eine Vorrichtung zur Überwachung des Gefäßzugangs eines Patienten, die z.B. in eine Dialysemaschine integriert sein kann, anzuschließen. Die elektrischen Anschlussstellen 5 sind zwischen zwei Knickkanten angeordnet. In Figur 6 ist eine Abdeckvorrichtung vollständig zusammengefaltet gezeigt, mit insgesamt fünf Knickkanten und den nach Faltung daraus entstehenden drei Faltentaschen dargestellt.

Eine Abdeckvorrichtung mit mindestens fünf Knickkanten weist zwei Faltentaschen auf, die sich bei Auseinanderziehen des beschichteten Papiers nach oben öffnen. Die mittlere Faltentasche öffnet sich nach unten in Richtung des Patienten. Das beschichtete Papier 1 ist zu Beginn der Behandlung, wenn es aus der Verpackung entnommen wird steril. Es wird dann zur Abdeckung des Patienten benutzt, die Oberfläche ist zum Umfeld exponiert und kann so kontaminiert werden, während das Schlauchsystem mit den Konnektoren in der untersten Faltentasche 10, die auch den Flüssigkeitssensor 2 enthält, geschützt ist. Müssen an den Konnektoren während der Behandlung Arbeitsschritte vorgenommen werden, muss das Schlauchsystem aus der Faltentasche entnommen und im Fall einer Abdeckungsvorrichtung wie z.B. in Figur 1 gezeigt, auf einem zur Krankenhausumgebung exponierten Bereich abgelegt werden. Die in Figur 6 gezeigte Abdeckungsvorrichtung weist eine Faltentasche ohne Flüssigkeitssensor 2 auf, die während der Behandlung zusammengefaltet bleiben und so geschützt bleiben kann. Müssen am Schlauchsystem Manipulationen vorgenommen werden, kann diese Faltentasche aufgefaltet werden und das Schlauchsystem auf dem geschützt verbliebenen beschichteten Papier 1 abgelegt werden. Kommt es bei der Manipulation zu Leckagen, wird kein Fehlalarm ausgelöst.

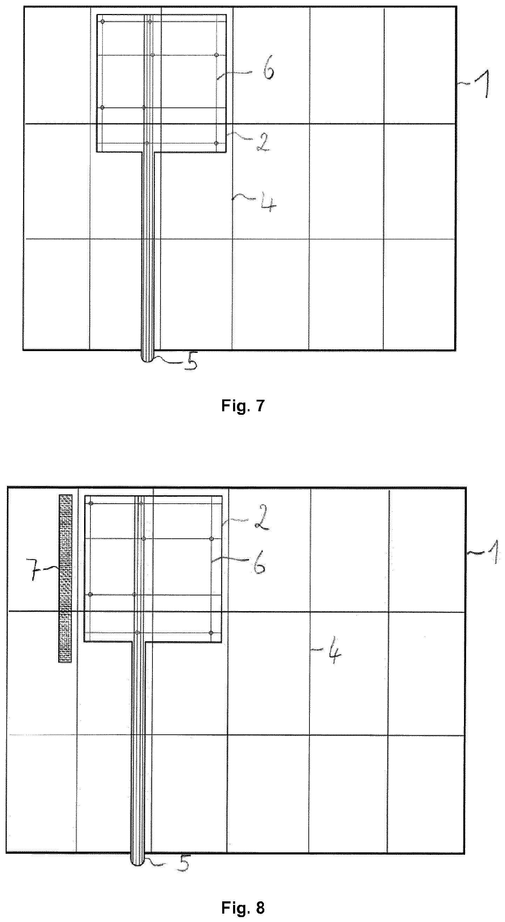

In Figur 7 ist die Aufsicht auf ein Flächengebilde 1 mit Flüssigkeitssensor 2 dargestellt. Der Flüssigkeitssensor 2 weist mehrere Leiterbahnen 6 auf, die z.B. wie dargestellt in das Gewebe der Flüssigkeitssensor 2 eingewebt sein können. An den mit Kreisen markierten Kreuzungspunkten berühren sich die leitfähigen Fäden und bilden auf diese Weise zwei Leiterschleifen. Die Anschlusskontakte 5 sind so gestaltet, dass sie mit einer Vorrichtung zur Überwachung des Gefäßzugangs verbunden werden können.

In Figur 8 ist die Aufsicht auf ein Flächengebilde 1 mit Flüssigkeitssensor 2 in aufgeklapptem Zustand dargestellt. Seitlich von der flüssigkeitssensitiven Fläche ist eine Barriere 7 angebracht. Sie besteht aus einem saugfähigen Material ohne Messfunktion. Flüssigkeitssensor 2 und Barriere 7 sind kontaktfrei angeordnet. Diese Barriere verhindert bei Manipulationen am Gefäßzugang oder den Konnektoren während oder vor der Behandlung, dass austretende Flüssigkeiten seitlich in die Faltung eindringen und in Kontakt mit der Flüssigkeitssensor 2 gelangen und so einen Fehlalarm auslösen.

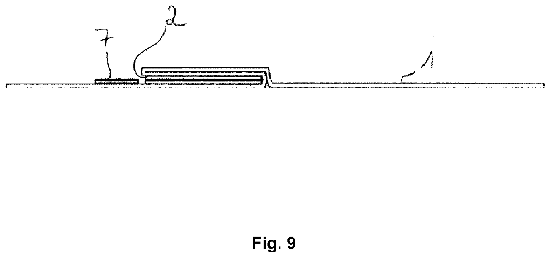

In Figur 9 ist ein Querschnitt des in Figur 9 beschriebenen beschichteten Papiers 1 gezeigt. Der Flüssigkeitssensor 2 ist eingeklappt.

## Patentansprüche

1. Abdeckungseinrichtung zur Abdeckung eines an einen Patienten anschließbaren Schlauchsystems (3), aufweisend ein Flächengebilde (1) mit einem Flüssigkeitssensor (2), **dadurch gekennzeichnet, dass** das Flächengebilde (1) eine erste Knickkante (4) aufweist, und der Flüssigkeitssensor (2) und die erste Knickkante (4) derart auf dem Flächengebilde (1) angeordnet sind, dass der Flüssigkeitssensor (2) bei einer Faltung des Flächengebildes (1) entlang der Knickkante (4) in einer sich durch die Faltung ergebenden Faltentasche angeordnet ist, und .
dass das Flächengebilde (1) eine zweite Knickkante (4') aufweist, die weitgehend parallel zur ersten Knickkante (4) verläuft und in Gegenrichtung zur ersten Knickkante (4) ausgebildet ist, wodurch sich bei Faltung des Flächengebildes (1) entlang der ersten und der zweiten Knickkante (4, 4') eine Zickzackfaltung mit zwei Faltentaschen ergibt und
dass die Unterseite des Flächengebildes flüssigkeitsundurchlässig ist.

2. Abdeckungseinrichtung nach Anspruch 1, **dadurch gekennzeichnet, dass** das Flächengebilde (1) zumindest vier parallel verlaufende Knickkanten (4, 4') aufweist, wobei benachbarte Knickkanten (4, 4') stets in entgegengesetzten Richtungen ausgebildet sind und sich bei der Faltung eine Zickzackfaltung und zumindest drei Faltentaschen ergeben.

3. Abdeckungseinrichtung nach zumindest einem der vorangehenden Ansprüche, **dadurch gekennzeichnet, dass** die sich bei Faltung ergebende Faltentasche breiter als ein abzudeckendes Schlauchsystem (3) ist.

4. Abdeckungseinrichtung nach zumindest einem der vorangehenden Ansprüche, **dadurch gekennzeichnet, dass** der Flüssigkeitssensor (2) höchstens auf einer Knickkante (4, 4') des Flächengebildes (1) liegt.

5. Abdeckungseinrichtung nach zumindest einem der vorangehenden Ansprüche, **dadurch gekennzeichnet, dass** der Flüssigkeitssensor (2) und das Flächengebilde (1) miteinander verbunden sind.

6. Abdeckungseinrichtung nach zumindest einem der Ansprüche 1 bis 4, **dadurch gekennzeichnet, dass** der Flüssigkeitssensor (2) in dem Flächengebilde (1) integriert ist.

7. Abdeckungseinrichtung nach zumindest einem der vorangehenden Ansprüche, **dadurch gekennzeichnet, dass** der Flüssigkeitssensor (2) eine elektrisch leitende Struktur (6) aufweist.

8. Abdeckungseinrichtung nach Anspruch 7, **dadurch gekennzeichnet, dass** die elektrisch leitende Struktur (6) in den Flüssigkeitssensor (2) eingewebt oder aufgedruckt ist.

9. Abdeckungseinrichtung nach zumindest einem der Ansprüche 7 oder 8, **dadurch gekennzeichnet, dass** die elektrisch leitende Struktur (6) Anschlusskontakte (5) zur Verbindung mit einer Vorrichtung zur Überwachung eines Zugangs zu einem Patienten aufweist.

10. Abdeckungseinrichtung nach zumindest einem der Ansprüche 1 bis 9, **dadurch gekennzeichnet, dass** das Flächengebilde (1) ein Textil, eine saugfähige Folie und/oder ein Vlies enthält.

11. Abdeckungseinrichtung nach zumindest einem der vorangehenden Ansprüche, **dadurch gekennzeichnet, dass** zumindest eine Seite des Flächengebildes (1) steril ist.

12. Abdeckungseinrichtung nach zumindest einem der vorangehenden Ansprüche, **dadurch gekennzeichnet, dass** am Rand des Flüssigkeitssensors (2) auf dem Flächengebilde (1) eine Barriere (7) aus einem saugfähigen Material ohne Messfunktion angebracht ist, um ein seitliches Eindringen von Flüssigkeit in die Faltentasche zu vermeiden.

13. System aufweisend eine Vorrichtung zur Überwachung eines Zugangs zu einem Patienten für eine Einrichtung, mit der über ein Schlauchsystem (3) eine Flüssigkeit einem Patienten zugeführt und/oder eine Flüssigkeit von dem Patienten abgeführt wird, insbesondere zur Überwachung
der Konnektoren des Schlauchsystems bei einer extrakorporalen Blutbehandlung, sowie eine damit verbundene Überwachungsvorrichtung mit einer Abdeckungseinrichtung nach einem der Ansprüche 1 bis 12.

## Claims

1. Covering device for covering a tubing system (3) that can be connected to a patient, comprising a sheet (1) with a liquid sensor (2), **characterized in that** the sheet (1) has a first bending edge (4), and the liquid sensor (2) and the first bending edge (4) are arranged on the sheet (1) in such a way that, when the sheet (1) is folded along the bending edge (4), the liquid sensor (2) is arranged in a gusseted pocket formed by the folding, and
**in that** the sheet (1) has a second bending edge (4'), which runs largely parallel to the first bending edge (4) and is formed in the opposite direction from the first bending edge (4) such that, when the sheet (1) is folded along the first and the second bending edges (4, 4'), a zigzag fold with two gusseted pockets is formed and
**in that** the underside of the sheet is impermeable to liquid.

2. Covering device according to Claim 1, **characterized in that** the sheet (1) has at least four parallel bending edges (4, 4'), wherein neighbouring bending edges (4, 4') are always formed in opposite directions and one zigzag fold and at least three gusseted pockets are formed by the folding.

3. Covering device according to one of the preceding claims, **characterized in that** the gusseted pocket formed by the folding is wider than a tubing system (3) to be covered.

4. Covering device according to at least one of the preceding claims, **characterized in that** the liquid sensor (2) lies at most on one bending edge (4, 4') of the sheet (1).

5. Covering device according to at least one of the preceding claims, **characterized in that** the liquid sensor (2) and the sheet (1) are connected to one another.

6. Covering device according to at least one of Claims 1 to 4, **characterized in that** the liquid sensor (2) is integrated in the sheet (1).

7. Covering device according to at least one of the preceding claims, **characterized in that** the liquid sensor (2) has an electrically conductive structure (6) .

8. Covering device according to Claim 7, **characterized in that** the electrically conductive structure (6) is woven into or printed on the liquid sensor (2).

9. Covering device according to at least one of Claims 7 and 8, **characterized in that** the electrically conductive structure (6) has terminal contacts (5) for connecting to a device for monitoring an access to a patient.

10. Covering device according to one of Claims 1 to 9, **characterized in that** the sheet (1) comprises a textile, an absorbent film and/or a nonwoven.

11. Covering device according to at least one of the preceding claims, **characterized in that** at least one side of the sheet (1) is sterile.

12. Covering device according to at least one of the preceding claims, **characterized in that** a barrier (7) of an absorbent material without a measuring function is attached to the sheet (1) at the edge of the liquid sensor (2) to prevent liquid from penetrating into the gusseted pocket at the side.

13. System comprising a device for monitoring an access to a patient for a device by which a liquid is supplied to a patient and/or a liquid is withdrawn from a patient through a tubing system (3), in particular for monitoring the connectors of the tubing system in an extracorporeal blood treatment, and also a monitoring device with a covering device according to one of Claims 1 to 12.

## Revendications

1. Dispositif de recouvrement destiné à recouvrir un système de tuyaux (3) pouvant être raccordé à un patient, présentant une structure plane (1) pourvue d'un détecteur de liquide (2), **caractérisé en ce que** la structure plane (1) possède un premier bord de pliage (4) et le détecteur de liquide (2) et le premier bord de pliage (4) sont disposés sur la structure plane (1) de telle sorte que le détecteur de liquide (2), lors d'un pliage de la structure plane (1) le long du premier bord de pliage (4), est disposé dans un sac à soufflets résultant du pliage, et
**en ce que** la structure plane (1) possède un deuxième bord de pliage (4'), qui suit un tracé en grande partie parallèle au premier bord de pliage (4) et qui est configuré en sens inverse du premier bord de pliage (4), moyennant quoi il se produit un pliage en zigzag avec deux sacs à soufflets lors du pliage de la structure plane (1) le long du premier et du deuxième bord de pliage (4, 4') et
**en ce que** le côté inférieur de la structure plane est imperméable aux liquides.

2. Dispositif de recouvrement selon la revendication 1, **caractérisé en ce que** la structure plane (1) possède au moins quatre bords de pliage (4, 4') qui suivent un tracé parallèle, les bords de pliage (4, 4') étant toujours formés dans des directions opposées et un pliage en zigzag ainsi qu'au moins trois sacs à soufflets se produisant lors du pliage.

3. Dispositif de recouvrement selon au moins l'une des revendications précédentes, **caractérisé en ce que** le sac à soufflets qui se produit lors du pliage est plus large qu'un système de tuyaux (3) à recouvrir.

4. Dispositif de recouvrement selon au moins l'une des revendications précédentes, **caractérisé en ce que** le détecteur de liquide (2) repose au maximum sur un bord de pliage (4, 4') de la structure plane (1).

5. Dispositif de recouvrement selon au moins l'une des revendications précédentes, **caractérisé en ce que** le détecteur de liquide (2) et la structure plane (1) sont reliés l'un à l'autre.

6. Dispositif de recouvrement selon au moins l'une des revendications 1 à 4, **caractérisé en ce que** le détecteur de liquide (2) est intégré dans la structure plane (1).

7. Dispositif de recouvrement selon au moins l'une des revendications précédentes, **caractérisé en ce que** le détecteur de liquide (2) possède une structure (6) électriquement conductrice.

8. Dispositif de recouvrement selon la revendication 7, **caractérisé en ce que** la structure (6) électriquement conductrice est incorporée par tissage dans le détecteur de liquide (2) ou imprimée sur celui-ci .

9. Dispositif de recouvrement selon au moins l'une des revendications 7 ou 8, **caractérisé en ce que** la structure (6) électriquement conductrice possède des contacts de raccordement (5) destinés à la liaison avec un arrangement servant à la surveillance d'un accès à un patient.

10. Dispositif de recouvrement selon au moins l'une des revendications 1 à 9, **caractérisé en ce que** la structure plane (1) contient un textile, un film absorbant et/ou un non-tissé.

11. Dispositif de recouvrement selon au moins l'une des revendications précédentes, **caractérisé en ce qu'**au moins un côté de la structure plane (1) est stérile.

12. Dispositif de recouvrement selon au moins l'une des revendications précédentes, **caractérisé en ce qu'**une barrière (7) constituée d'un matériau absorbant sans fonction de mesure est appliquée sur la structure plane (1) au niveau du bord du détecteur de liquide (2) afin d'empêcher une pénétration latérale de liquide dans le sac à soufflets.

13. Système possédant un arrangement destiné à surveiller un accès à un patient pour un dispositif, avec lequel un liquide est acheminé à un patient et/ou un liquide est évacué du patient par le biais d'un système de tuyaux (3), notamment destiné à surveiller les connecteurs du système de tuyaux lors d'un traitement du sang extracorporel, ainsi qu'un arrangement de surveillance relié à celui-ci et pourvu d'un dispositif de recouvrement selon l'une des revendications 1 à 12.
